# EUROPEAN PATENT APPLICATION

(11) **EP 0 875 155 A1**
(43) Date of publication of application: **04.11.1998**
(21) Application number: 97201309.8
(22) Date of filing: 01.05.1997
(51) Int. Cl.: A23L 1/30, A23L 1/305, A61K 31/195, A23L 1/09, A61K 31/70, A61K 35/20, A61K 31/715

(54) **Peri-operative drink**

(71) Applicant: N.V. Nutricia, 2700 MA Zoetermeer (NL)
(72) Inventor: Hofman, Zandrie, NL-2727 HR Zoetermeer (NL); Hageman, Robert Johan Joseph, NL-2742 EV Waddinxveen (NL)
(74) Representative: de Bruijn, Leendert C.

(57) **Abstract**

A liquid nutritional composition for peri-operative use is described which contains, per 400 ml, 5-130 g of soluble carbohydrates and 1-30 g of glutamine or a glutamine precursor calculated as glutamine. The carbohydrates preferably consist of at least 75 % by weight of polysaccharides. The composition can further contain 0.5-20 g of one or more amino acids selected from arginine, lysine, ornithine, histidine, and other specific amino acids per 400 ml. The contents of natural fats and insoluble proteins are low.

## Description

The invention pertains to a liquid nutritional composition for administration shortly before and after surgery and other conditions characterised by a demand for rapid gastric emptying and reversing catabolic processes and/or maintaining anabolism.

Before a medical operation, such as surgery, the patient is usually subjected to fasting for at least 8 hours, up to 24 hours, for reasons of safety with regard to anaesthesia and for preventing regurgitation of the stomach content and aspiration. This fasting, however, has undesired consequences for the patient. Firstly, the patient will suffer bad well-being as a result of hunger and thirst. Secondly, and more importantly, the patient's metabolism will be disturbed, and may even become catabolic. Such a catabolic state is highly undesired, since it results in loss of body weight and in energetically inefficient degradation of proteins, including proteins that are vital for local healing after the operation.

Therefore, there is a need for feeding the patient before surgery as long as possible, for example up to 90 minutes before operation, so as to keep the metabolism anabolic, without causing problems of anaesthesia and emptying of the stomach.

EP-A-564511 discloses a beverage for preoperative intake consisting of an aqueous solution which is hypotonic (250-295 mOsm/kg) and contains 8-20 g of carbohydrates per 100 ml. The carbohydrates consist of 10-30 % by weight of monosaccharides, 10-30 % by weight of disaccharides and the remainder (40-80 % by weight) polysaccharides (dextrins). No other ingredients are contemplated, except sodium or potassium chloride for adjusting the required osmolality and sodium hydroxide for adjusting the pH (5.6-6.8) and preservatives. The product was designed for rapidly passing the stomach and triggering hormonal response (insulin). It can be given to the patient until about 1 hour before operation.

EP-A-674902 discloses a dietary supplement to be given before operation, containing arginine or omithine and ω-3 poly-unsaturated fatty acids (PUFA's), in addition to other ingredients including ω-6 PUFA's, proteins, carbohydrates, fats, nucleobases, salts, vitamins and the like. The product should stimulate the immune system after operation. It is not intended to be used shortly before operation, and the protein and fat content will ensure that the stomach residence time will be long.

Similarly, EP-A-704212 teaches the use of protected ω-3 PUFa's in a preoperative diet for minimising hepatic injury following surgery. Again, the product cannot be used shortly before surgery because of the presence of proteins and fats.

The preoperative use of arginine is also proposed in WO 96/36327. Its purpose is to improve micro-circulatory hypoperfusion. It is to be administered between 3 and 10 days before surgery.

It was found that a liquid composition which is largely free of common fats and intact insoluble proteins, and contains soluble carbohydrates and glutamine, can be given to patients until shortly (e.g. 90 minutes) before operation, but also shortly after operation, without negative consequences for the patient during operation, and is effective in maintaining an anabolic metabolism and in particular in supporting protein synthesis and immune functions, so as to accelerate healing after the operation. The same composition was furthermore found to be suitable for feeding persons that require rapid gastric emptying and maintenance of an anabolic state, such as pregnant women just before delivery of the baby, persons that are in a state of high physical exercise, for example during sports and during certain phases in cancer treatment.

The composition according to the invention is defined in the appending claims.

The soluble carbohydrates preferably contain a majority of polysaccharides (i.e. more than 50 % by weight). In particular the weight percentage of polysaccharides is at least 75 % or even at least 80 %. The polysaccharides are meant as any water-soluble saccharide containing more than two monosaccharide units and may be constituted by dextrins, maltodextrins and the like. A preferred monosaccharide is fructose, as it induces a delayed insulin response, and does not impart gastric emptying. Fructose is preferably present at a level of 1-10 g per 400 ml. Preferably no substantial amounts of other mono- and disaccharides are present in the composition; in particular the glucose content is less than 5 g per 400 ml.

Glutamine is present at a level of 1-30 g per 400 ml, preferably from 5 to 25 g / 400 ml. It may be present as such, but it may also be present as a precursor of glutamine, like glutamine-rich peptides, such as glutaminylglycine, glutamylglutamine or alanylglutamine or mixtures thereof or other glutamine-containing sources. Also water-soluble hydrolysates of glutamin-rich proteins (like wheat-gluten protein) can be used as an ingredient, if they are low in salt content (ash). Demineralisation preferably occurs before spray-drying using known methods. In case of a glutamine precursor, its level is based on its glutamine content. A protein hydrolysate containing 20-35 wt.% of glutamine (as only glutamine source), such as for example derived from wheat gluten, can be used at a level of 5-70 g per 400 ml.

It is preferred that the composition also contains 0.5-20, especially 2-15 g / 400 ml of other amino acids such as arginine, ornithine, lysine or histidine, or their metabolic precursors. Preferably these amino acids are included at a level of 0.5-15 g, especially 2-8 g / 400 ml. Among these, arginine is especially preferred.

The composition according to the invention advantageously also contains growth factors, e.g. derived from a liquid dairy product containing these. Preferably colostrum or a fraction thereof is used. The liquid dairy product may represent 1-100 ml per 400 ml of the composition. It can be used e.g. as a spray-dried defatted colostrum whey at a rate of 0.1-10 g whey powder. If a growth factor isolate from colostrum is used, 1-1000 mg thereof can be used.

The composition also preferably comprises 0.2-8 g, especially 0.3-3.0 g of methionine and/or cysteine or their metabolic equivalents (such as N-acetylcysteine) per 400 ml. Further desirable amino acids comprise glycine, serine, alanine and glutamate, which may each be present at 0.2-20 especially 0.5-8 g / 400 ml.

Furthermore the composition may contain vitamins, trace elements, minerals and other components, for example those given below or their metabolic equivalents.

| | *preferred amount per 400 g* |
|---|---|
| * Trace elements, minerals | |
| magnesium | 60-400 mg |
| zinc | 4-40 mg |
| copper | 0.6-20 mg |
| selenium | 20-300 µg |
| manganese | 2-40 mg |
| chromium | 20-400 µg |

| * Vitamins | |
|---|---|
| folic acid | 80-1000 µg |
| pyridoxine | 0.7-18 mg |
| cyanocobalamine | 0.8-16 µg |
| vitamin C | 20-400 mg |
| vitamin D | 0.5-20 µg |
| thiamine | 0.4-6 mg |
| riboflavine | 0.5-7 mg |
| nicotinamide | 5-75 mg NE |
| biotine | 30-400 µg |

| * Other components | |
|---|---|
| nucleotides | 2-500 mg yeast extract |
| lecithin or other phospholipids, medium-chain triglyderides (MCT's) creatine, pyruvate, carotenoids, flavonoids | |

Also a blend of antioxidants like selenium, ascorbic acid, tocopherols, carotenoids and flavonols can be included advantageously. Preferably' the composition is low in fats and proteins. In particular it contains less than 2 % by weight of fat. The fat preferably contains less 2 %, more preferably less than 1 % by weight of lauric and myristic fatty acids with respect to the total fatty acids. Especially if the fat is present at a level of more than 0.5 %, it advantageously consists of at least 50 % by weight of medium-chain fatty acids (C₈ and C₁₀), lecithins, phospholipids or structured fats. The fat further preferably contains 3-60 wt.% of long-chain polyunsaturated fatty acids. The preferred fat level is 0.3-1.5 wt.%.

The composition may contain up to 4 wt.% of soluble proteins, such as whey proteins, preferably 0.5-2 wt.% (soluble is understood to mean soluble at pH 2.0 to 7.5). It contains less than 1 wt.%, preferably less than 0.5 wt.%, more preferably less than 0.1 wt.% of insoluble (at pH 3) intact proteins, such as caseins. The osmolality should not be too high although hypotonicity is not absolutely necessary. The osmolality may e.g. be between 250 and 450, especially between 300 and 400 mOsm/kg. The pH of the composition is between 4.0 and 8.0, preferably between 5.5 and 7.5. If necessary the desired can be adjusted by addition of a buffering agent such as phosphate or especially citrate. The energy density of the composition is not more than 1.3, and preferably less than 1.0 kcal per ml of (reconstituted) liquid.

The composition is preferably a liquid composition which can be administered as such. It may alternatively be a dried, or dry, composition containing all the desired ingredients, which can be reconstituted before use by the addition of a specified amount of water. The composition may be prepared by methods known per se, which may comprise dissolving, mixing, buffering, homogenising, pasteurising, spray-drying, and the like. It can be administered enterally e.g. by drinking or tube-feeding. It can be administered in an amount of 50 to 600 ml - depending on the patient - up to 1 hour before operation, if appropriate preceded by similar or higher amounts longer before the operation. From half an hour onwards after the operation, similar amounts can be given according to the needs of the patient. For other conditions, such as delivery or other physical exertion, adapted amounts can be given.

### Example 1

A liquid perioperative composition was prepared by mixing the following components:

| *Ingredient:* | *Amount per 5000 l* |
|---|---|
| Maltodextrin DE19 | 625 kg |
| Wheat protein hydrolysate | 375 kg |
| L-Arginine | 37.5 kg |
| L-Lysine.HCl | 25 kg |
| L-Methionine | 6.25 kg |
| Bovine colostrum whey protein powder | 7.5 kg |
| Ascorbic acid | 625 g |
| Pyridoxamine | 25 g |
| Folic acid | 2.5 g |
| Niacine | 250 g NE |
| Biotine | 1.25 g |
| Trimagnesium dicitrate | 4.4 kg |

The energy density of this composition was about 0.86 kcal/ml. The glutamine content was about 9 g / 400 ml and the pH was 6.7.

### Example 2

A liquid perioperative composition was prepared by mixing the following components:

| *Ingredient:* | *Amount per 5000 l* |
|---|---|
| Maltodextrin DE19 | 710 kg |
| Fructose | 40 kg |
| Alanylglutamine | 310 kg |

The energy density of this composition was about 0.85 kcal/ml.

## Claims

1. A liquid nutritional composition for enteral peri-operative use, containing, per 400 ml, 5-130 g of soluble carbohydrates and 1-30 g of glutamine or a glutamine precursor calculated as glutamine.

2. A composition according to claim 1, containing, per 400 ml, 15-100 g, preferably 24-80 g of soluble carbohydrates, the carbohydrates preferably consisting of at least 75 % by weight of polysaccharides, and optionally 1-40 g of fructose.

3. A composition according to claim 1 or 2, containing 5-25 g of glutamine or glutamine precursor per 400 ml.

4. A composition according to any one of claims 1-3, further containing 0.5-20 g of one or more amino acids selected from arginine, lysine, ornithine and histidine, preferably comprising 0.5-15 g, most preferably 2-8 g of arginine, per 400 ml.

5. A composition according to any one of claims 1-4, further containing 0.2-8 g of a sulphur-containing amino acid selected from methionine and cysteine, and/or 0.2-20 g of each of glycine, alanine, serine and/or glutamate per 400 ml.

6. A composition according to any one of claims 1-5, further containing, per 400 ml, 1-100 ml of liquid dairy product rich in growth factors, especially containing defatted colostrum corresponding to 0.1-10 g of whey protein powder.

7. A composition according to any one of claims 1-6, having a fat content of 0.3-1.5 % by weight of the total composition, the fat preferably comprising less than 1 % of C₁₂ and C₁₄ fatty acids by weight of the total fatty acids.

8. A composition according to any one of claims 1-7, having a content of proteins which are not soluble at pH 2.0-7.5 of less than 0.5 % by weight of the total composition.

9. A composition according to any one of claims 1-8, having an osmolality of below 450 mOsm/kg, and an energy density below 1.0 kcal/ml.

10. A dried composition to be reconstituted to a liquid composition according to any one of claims 1-9.
